Europäisches Patentamt

European Patent Office

Office européen des brevets

(19)

(11) Veröffentlichungsnummer: **0 260 550 B1**

# EUROPÄISCHE PATENTSCHRIFT

(12)

(45) Veröffentlichungstag der Patentschrift: **07.08.91**

(51) Int. Cl.⁵: **A61B 17/22, A61B 6/04**

(21) Anmeldenummer: **87112975.5**

(22) Anmeldetag: **04.09.87**

(54) **Lithotripsie-Arbeitsplatz.**

(30) Priorität: **19.09.86 DE 3631956**

(43) Veröffentlichungstag der Anmeldung:
**23.03.88 Patentblatt 88/12**

(45) Bekanntmachung des Hinweises auf die
Patenterteilung:
**07.08.91 Patentblatt 91/32**

(84) Benannte Vertragsstaaten:
**DE FR NL**

(56) Entgegenhaltungen:
**EP-A- 0 168 559**
**DE-A- 3 330 552**
**DE-U- 8 528 785**

(73) Patentinhaber: **Siemens Aktiengesellschaft**
**Wittelsbacherplatz 2**
**W-8000 München 2(DE)**

(72) Erfinder: **Grasser, Franz, Dipl.-Ing. (FH)**
**Neuwiesenstrasse 27**
**W-8557 Eggolsheim(DE)**
Erfinder: **Oppelt, Sylvester, Dipl.-Ing. (FH)**
**Greiffenbergstrasse 51**
**W-8600 Bamberg(DE)**

EP 0 260 550 B1

Rank Xerox (UK) Business Services

## Beschreibung

Die Erfindung betrifft einen Lithotripsie-Arbeitsplatz mit einem durch eine Steuervorrichtung dreidimensional verstellbaren Patientenlagerungstisch, unter dem mindestens ein Stoßwellengenerator für die Zertrümmerung von im Körper eines Patienten befindlichen und im Fokus des Stoßwellengenerators positionierten Objekten angeordnet ist, mit einer Röntgenuntersuchungseinrichtung zur Ortung dieser Objekte mit einer Röntgeneinheit, die den Patienten unter unterschiedlichen Winkeln durchstrahlt, und mit einer Röntgen-Fernsehaufnahmevorrichtung.

In dem DE-GM 85 28 785 ist ein derartiger Lithotripsie-Arbeitsplatz beschrieben, der zwei von einer Parkposition in eine Betriebsposition schwenkbare Stoßwellengeneratoren enthält. Zur Ortung ist der Arbeitsplatz mit einer Röntgendiagnostikeinrichtung mit zwei Röntgenstrahlenquellen zur Erzeugung zweier sich schneidender Röntgenstrahlenbündel und zwei Bildverstärker-Fernsehketten zur Darstellung der Röntgenbilder ausgerüstet. Die beiden Röntgenstrahlenbündel schneiden sich in einem Isozentrum, auf das die Stoßwellengeneratoren in Betriebsstellung fokussiert sind.

Das Einschwenken der Stoßwellengeneratoren erfolgt durch eine Aussparung in dem Patientenlagerungstisch, so daß eine Ankopplung des Stoßwellengenerators nur in bestimmten Stellungen des Patientenlagerungstisches erfolgen kann. Ist nunmehr das Konkrement durch die Röntgenuntersuchungsvorrichtung erkannt und durch Verschiebung des Patientenlagerungstisches in das Isozentrum bewegt, so kann bei entsprechender falscher Lagerung des Patienten eine Ankopplung deshalb nicht erfolgen, weil die Aussparung in dem Patientenlagerungstisch außerhalb des Bereiches der Stoßwellengeneratoren liegt.

Die Erfindung geht von der Aufgabe aus, einen Lithotripsie-Arbeitsplatz der eingangs genannten Art zu schaffen, der es ermöglicht, aufgrund der Röntgenbilder der Röntgenuntersuchungseinrichtung festzustellen, ob durch Verschieben des Patientenlagerungstisches eine Ankopplung möglich ist oder ob der Patient verlagert werden muß.

Die Aufgabe wird erfindungsgemäß dadurch gelöst, daß die Steuervorrichtung mit einer Erfassungsschaltung für die aktuelle Tischposition verbunden ist, an der eine Rechenschaltung zur Ermittlung der Bereichsgrenzen der Ankoppelmöglichkeiten des Stoßwellengenerators angeschlossen ist, und daß die Rechenschaltung mit einem Signalgenerator verbunden ist, der eine die Bereichsgrenzen kennzeichnende Markierung in das Videosignal der Fernsehaufnahmeeinrichtung einblendet. Dadurch kann die Untersuchungsperson aufgrund des Monitorbildes sofort erkennen, ob eine Ankopplung der Stoßwellengeneratoren möglich ist. Liegt das Objekt außerhalb dieser Bereichsgrenzen, so muß zuerst der Patient umgelagert werden.

Die Stellung des Isozentrums in bezug auf den Patientenlagerungstisch und dessen Öffnung lassen sich aus dem Monitorbild erkennen, wenn der Signalgenerator derart ausgebildet ist, daß er eine Einblendung eines die Mitte des Monitorbildes kennzeichnenden Fadenkreuzes in das Videosignal bewirkt.

Die Erfindung ist nachfolgend anhand eines in der Zeichnung dargestellten Ausführungsbeispieles näher erläutert. Es zeigen:

Fig. 1  einen erfindungsgemäßen Lithotripsie-Arbeitsplatz und

Fig. 2  ein Beispiel eines auf dem Monitor dargestellten Röntgenbildes mit Einblendungen nach der Erfindung.

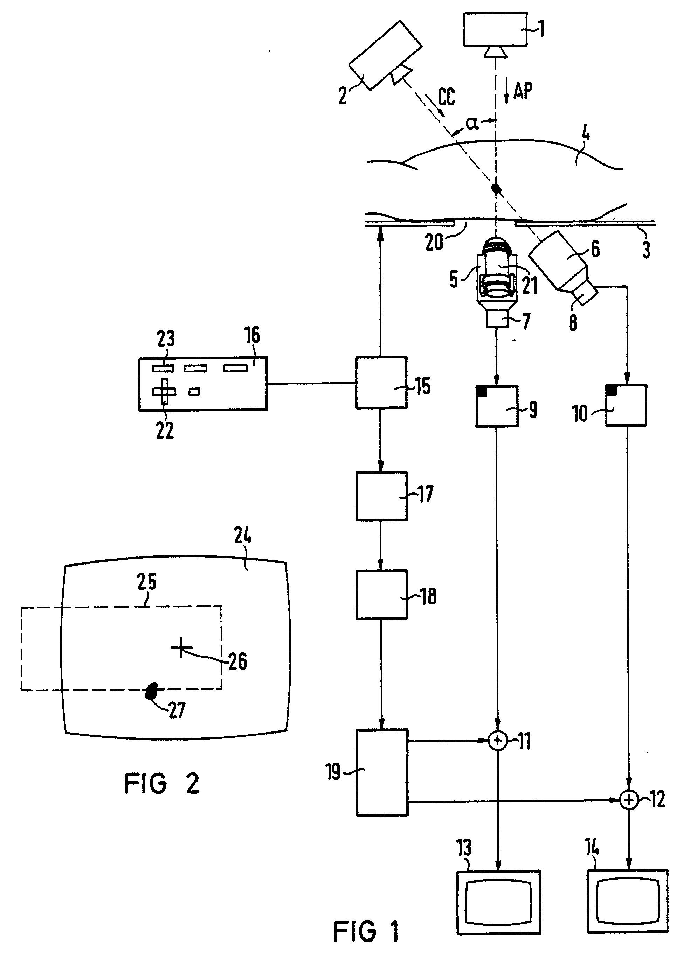

In der Figur 1 ist ein Lithotripsie-Arbeitsplatz mit einer Röntgendiagnostikeinrichtung dargestellt, die zwei Röntgenröhren 1 und 2 aufweist, die Röntgenstrahlenbündel erzeugen, die einen auf einem Patientenlagerungstisch 3 befindlichen Patienten 4 durchdringen und auf die Eingangsleuchtschirme von Röntgenbildverstärkern 5 und 6 fallen. Die Röntgenröhre 1 und der Röntgenbildverstärker 5 können dabei derart angeordnet sein, daß der Zentralstrahl des Röntgenstrahlenbündels der Röntgenröhre 1 senkrecht auf den Patienten fällt (a.p.-Projektion). Die Röntgenröhre 2 und der Röntgenbildverstärker 6 sind beispielsweise derart schräg angeordnet, daß der Zentralstrahl der Röntgenröhre 2 den Zentralstrahl der Röntgenröhre 1 im Isozentrum innerhalb des Patienten 4 unter einem Winkel alpha von beispielsweise 45° schneidet (c.c.-Projektion). Dadurch erhält man Durchleuchtungsbilder aus zwei verschiedenen Projektionsrichtungen, so daß man den Patienten 4 durch den Patientenlagerungstisch 3 derart dreidimensional verschieben kann, daß sich beispielsweise ein Nierenstein in dem Isozentrum befindet.

In dem Patientenlagerungstisch 3 ist eine Öffnung 20 angebracht, durch die Stoßwellengeneratoren 21 aus ihrer Parkposition in die Betriebsstellung geschwenkt werden können, bis sie mit einer Membran an der Hautoberfläche des Patienten 4 anliegen. Mit der Schwenkung wird gleichzeitig der Fokus der Stoßwellengeneratoren 21 auf das Isozentrum ausgerichtet, so daß in der Betriebsstellung die Stoßwellenbehandlung erfolgen und das Objekt, beispielsweise ein Nierenstein, zertrümmert werden kann.

Die Ausgangssignale der an den Röntgenbildverstärkern 5 und 6 angekoppelten Fernsehkameras 7 und 8 können in zwei Bildspeichern 9 und 10 eingelesen werden. Die Ausgangssignale der Bildspeicher 9 und 10 sind mit den Eingängen von Additionsstufen 11 und 12 verbunden, an denen je ein Monitor 13 und 14 zur Wiedergabe der Röntgenbilder angeschlossen ist.

Nach erfolgter Durchleuchtung durch die Röntgenröhren 1 und 2 werden die Röntgenstrahlenbilder von den Bildwandlern, den Bildverstärkern 5 und 6 sowie den angekoppelten Fernsehkameras 7 und 8, in Bildsignale umgewandelt, die in den Bildspeichern 9 und 10 eingespeichert werden. Den Röntgenstrahlenbildern wird, wie noch erläutert, in den Additionsstufen 11 und 12 ein Signal zugemischt, so daß auf den Monitoren 13 und 14 die Röntgenstrahlenbilder mit einer Einblendung dargestellt werden.

Zur dreidimensionalen Verschiebung des Patientenlagerungstisches 3 ist dieser mit einer Steuervorrichtung 15 für dessen motorische Verstellung verbunden. Über ein Bedienpult 16 kann der Patientenlagerungstisch 3 verschoben werden, wobei die aktuelle Position der drei Raumkoordinaten durch Anzeigemittel 23 auf dem Bedienpult 16 erkennbar ist. An der Steuervorrichtung 15 ist eine Erfassungsschaltung 17 angeschlossen, die die aktuelle Tischposition ermittelt. Aus diesen Werten berechnet eine an der Erfassungsschaltung 17 angeschlossene Rechenschaltung 18 aufgrund in der Rechenschaltung enthaltener Daten über die Öffnung 20 im Patientenlagerungstisch 3 die Bereichsgrenzen der Ankoppelmöglichkeiten der Stoßwellengeneratoren 21. Dieser Rechenwert der Rechenschaltung 18 wird einem Signalgenerator 19 zugeführt, der aufgrund dieser Werte Videosignale erzeugt, die eine Einblendung einer die Bereichsgrenzen kennzeichnenden Markierung 25 in das Monitorbild 24 bewirken. Ebenfalls wird vom Signalgenerator 19 ein Fadenkreuz 26 in die Mitte der Monitorbilder 24 eingeblendet, das das Isozentrum kennzeichnet.

In der Figur 2 ist ein derartiges Monitorbild 24 dargestellt, wobei nur die für die Erfindung wesentlichen Merkmale wiedergegeben wurden. In dem Monitorbild 24 ist gestrichelt die durch die Öffnung 20 kennzeichnende Bereichsgrenze als Markierung 25 dargestellt. Das Fadenkreuz 26 gibt die Lage des Isozentrums in der Mitte des Monitorbildes 24 an. Das Objekt 27, beispielsweise ein Nierenstein, ist ebenfalls in dem Monitorbild 24 zu erkennen. Es liegt auf den Markierungen 25 der Bereichsgrenzen und außerhalb des durch das Fadenkreuz 26 gekennzeichneten Isozentrums. Daraus erkennt die Untersuchungsperson, daß eine Ankopplung des Stoßwellengenerators 21 nur schlecht zu erreichen ist. Es muß vor der Behandlung der Patient 4 auf dem Patientenlagerungstisch 3 geringfügig umgelagert werden. Eine Umlagerung des Patienten 4 wäre auch erforderlich, wenn das Objekt 27 außerhalb dieser Markierungen 25 läge. Erscheint das Objekt 27 nach der Umlagerung des Patienten 4 innerhalb der die Bereichsgrenzen kennzeichnenden Markierung 25, so kann eine Ankopplung erfolgen, wenn der Patientenlagerungstisch 3 derart verschoben wird, daß sich das Objekt 27 im durch das Fadenkreuz 26 gekennzeichneten Isozentrum befindet. Dies kann in allen drei Dimensionen durch die Bedienelemente 22 des Bedienpultes 16 erfolgen, bis in beiden Bildern 24 der Monitore 13 und 14 das Fadenkreuz 26 und das Objekt 27 zur Deckung gebracht werden.

Als weitere Möglichkeit kann die Rechenschaltung 18 auch mit einer nicht dargestellten Schaltung zur Schwenkung der Stoßwellengeneratoren 21 aus ihrer Parkposition in ihre Betriebsstellung verbunden sein. Da die Rechenschaltung 18 die Bereichsgrenzen der Ankoppelmöglichkeit der Stoßwellengeneratoren 21 ermittelt, kann der Schaltung ein Signal zugeführt werden, das diese dann sperrt, so daß ein Schwenken der Stoßwellengeneratoren 21 in ihre Betriebsstellung unterbunden wird, wenn sie sich nicht unterhalb der Öffnung 20 in dem Patientenlagerungstisch 3 befinden. Dadurch wird eine Beschädigung der Stoßwellengeneratoren 21 sicher vermieden.

## Patentansprüche

1.  Lithotripsie-Arbeitsplatz mit einem durch eine Steuervorrichtung (15) dreidimensional verstellbaren Patientenlagerungstisch (3), unter dem mindestens ein Stoßwellengenerator (21) für die Zertrümmerung von im Körper eines Patienten (4) befindlichen und im Fokus des Stoßwellengenerators (21) positionierten Objekten (27) angeordnet ist, mit einer Röntgenuntersuchungseinrichtung (1, 2, 5 bis 14) zur Ortung dieser Objekte (27) mit einer Röntgeneinheit (1, 2), die den Patienten (4) unter unterschiedlichen Winkeln durchstrahlt, und mit einer Röntgen-Fernsehaufnahmevorrichtung (5 bis 10), **dadurch gekennzeichnet,** daß die Steuervorrichtung (15) mit einer Erfassungsschaltung (17) für die aktuelle Tischposition verbunden ist, an der eine Rechenschaltung (18) zur Ermittlung der Bereichsgrenzen der Ankoppelmöglichkeiten des Stoßwellengenerators (21) angeschlossen ist, und daß die Rechenschaltung (18) mit einem Signalgenerator (19) verbunden ist, der eine die Bereichsgrenzen kennzeichnende Markierung (25) in das Videosignal der Fernsehaufnahmevorrichtung (5 bis 10) einblendet.

2.  Lithotripsie-Arbeitsplatz nach Anspruch 1, **dadurch gekennzeichnet,** daß der Signalgenerator (19) derart ausgebildet ist, daß er eine Einblendung eines die Mitte des Monitorbildes (24) kennzeichnenden Fadenkreuzes (26) in das Videosignal bewirkt.

## Claims

1. Lithotripsy operating station having a patient positioning table (3) which can be adjusted in three-dimensional manner by means of a control device (15), beneath which table at least one shock-wave generator (21) is arranged for the disintegration of objects (27) located in the body of a patient (4) and positioned in the focus of the shock-wave generator (21), having an X-ray examination device (1, 2, 5 to 14) for the location of these objects (27) with an X-ray unit (1, 2) by which radiation at various angles penetrates the patient (4), and having an X-ray television pickup device (5 to 10), characterized in that the control device (15) is connected to a detection circuit (17) for the actual table position, to which a computer circuit (18) is attached for the determination of the range limits of the coupling possibilities of the shock-wave generator (21), and in that the computer circuit (18) is connected to a signal generator (19) which mixes an indication (25), characterizing the range limits, into the video signal of the television pickup device (5 to 10).

2. Lithotripsy operating station according to claim 1, characterized in that the signal generator (19) is constructed in such a way that it effects mixing of a crosshair (26), characterizing the centre of the monitor image (24), into the video signal.

## Revendications

1. Poste de travail de lithotritie comportant un plateau (3) formant couchette pour patient, qui est déplaçable dans trois dimensions au moyen d'un dispositif de commande (15) et au-dessous duquel est disposé au moins un générateur d'ondes de choc (21) servant à fragmenter des objets (27) situés dans le corps d'un patient (4) et positionnés au foyer du générateur d'ondes de choc (21), un dispositif de radiodiagnostic (1,2,5 à 14) servant à localiser ces objets (27) et comportant une unité radiologique (1,2) qui réalise une irradiation du patient (4) sous différents angles, et un dispositif de röntgen-télévision (5 à 10), caractérisé par le fait que le dispositif de commande (15) est raccordé à un circuit (17) de détection de la position actuelle de la table, à laquelle est raccordé un circuit de calcul (18) servant à déterminer les limites de zone des possibilités d'accouplement du générateur d'ondes de choc (21) et que le circuit de calcul (18) est raccordé à un générateur de signaux (19), qui insère une marque (25) caractérisant les limites de zone, dans le signal vidéo du dispositif de röntgen-télévision (5 à 10).

2. Poste de travail de lithotritie suivant la revendication 1, caractérisé par le fait que le générateur de signaux (19) est agencé de manière à insérer un réticule (26) caractérisant le centre de l'image (24) d'un moniteur, dans le signal vidéo.

FIG 2

FIG 1